Europäisches Patentamt

European Patent Office

Office européen des brevets

(19)

(11) Veröffentlichungsnummer: **0 191 071**
**B1**

(12) **EUROPÄISCHE PATENTSCHRIFT**

(45) Veröffentlichungstag der Patentschrift: **18.04.90**

(51) Int. Cl.⁵: **A 61 M 1/10,** F 04 B 43/10

(21) Anmeldenummer: **85904103.0**

(22) Anmeldetag: **05.08.85**

(86) Internationale Anmeldenummer:
**PCT/EP85/00393**

(87) Internationale Veröffentlichungsnummer:
**WO 86/01115 27.02.86 Gazette 86/05**

(54) **PUMPVORRICHTUNG ZUR FÖRDERUNG VON GEGEN MECHANISCHE BEANSPRUCHUNGEN HOCHEMPFINDLICHE FLÜSSIGKEITEN.**

(30) Priorität: **04.08.84 DE 3428828**

(43) Veröffentlichungstag der Anmeldung:
**20.08.86 Patentblatt 86/34**

(45) Bekanntmachung des Hinweises auf die Patenterteilung:
**18.04.90 Patentblatt 90/16**

(84) Benannte Vertragsstaaten:
**AT BE CH DE FR GB IT LI LU NL SE**

(56) Entgegenhaltungen:
**DE-A-2 343 042**
**DE-A-2 634 238**
**DE-B-1 226 740**
**US-A-4 222 127**

(73) Patentinhaber: BRAUN, Karl-Theo
Kolpingstrasse 58a
D-6652 Bexbach (DE)

(72) Erfinder: BRAUN, Karl-Theo
Kolpingstrasse 58a
D-6652 Bexbach (DE)

(74) Vertreter: Patentanwälte Zellentin & Partner
Zweibrückenstrasse 15
D-8000 München 2 (DE)

Courier Press, Leamington Spa, England.

## Beschreibung

Die Erfindung betrifft eine Pumpvorrichtung zur Förderung von gegen mechanische Beanspruchungen hochempfindlichen Flüssigkeiten, mit einem festen Gehäuse, das einen flexiblen Wandteil aufweist und einen abgedichteten Hohlraum veränderlichen Volumens umgibt, der eine Arbeitsflüssigkeit enthält und über eine Anschlußleitung mit einer Pumpe verbunden ist, und mit einer flüssigkeitsdichten, mit Zu- und Abflüssen mit Ventilen versehenen Förderkammer für die Förderflüssigkeit, die mindestens einen flexiblen Wandteil aufweist, wobei die flexiblen Wände des Gehäuses und der Förderkammer einander gegenüberliegend in flächigen Berührungskontakt bringbar angeordnet sind und als gegenseitige luftdichte Dichtungen gestaltet sind.

Menschliches Blut stellt eine gegen mechanische Beanspruchungen hochempfindliche Flüssigkeit dar, die z.B. unter Quetschbelastungen leicht zu einer permanenten Hämolyse- und Gerinnungsrate neigt.

Die Erfindung kann mit besonderem Vorteil in Blutbehandlungsvorrichtungen, wie Hämodialyse-, Ultrafiltrations-, Hämofiltrations- und Plasmaseparationsvorrichtungen eingesetzt werden.

Eine Pumpvorrichtung der eingangs genannten Art ist aus der US-A-4 222 127 bekannt. Diese bekannte Pumpvorrichtung wird als Herz eingesetzt, ist mit Kugelrückschlagventilen versehen und weist zwischen den flexiblen Wänden Lufträume auf. Mit dieser Pumpenvorrichtung ist eine exakt definierbare Volumendosierung nicht möglich, da die Rückschlagventile erst dann funktionieren, wenn bereits eine Strömung besteht und durch die komprimierbaren Luftkammern entstehen entsprechend den jeweiligen Druckverhältnissen andere Volumen.

Aus der DE-A1-23 43 042 ist eine Kolbenschlauchmembranpumpe bekannt, bei der eine Innenschlauchmembran konzentrisch in einer Außenschlauchmembran angeordnet ist, wobei sich zwischen den beiden Schlauchmembranen ein Hohlraum befindet, der einen Ausgang ins Freie aufweist. Die Ein- und Ausgänge sind über Kugelventile verschließbar. Bei dieser Kolbenschlauchmembranpumpe bestehen die gleichen Probleme wie sie oben geschildert sind.

Aus der DE-C-12 26 740 ist ein Membranverdichter bekannt, der zwei aufeinander liegende Membranen aufweist, wobei der Zwischenraum zwischen den Membranen an ein Vakuumgefäß mit einem Vakuummeter angeschlossen ist, das als Anzeigevorrichtung für einen Membranbruch dient.

Aus Sterilitätsgründen müssen die Fördervorrichtungen, die für den extrakorporalen Bluttransport zu Filtern oder Austauschmembranen oder dergleichen eingesetzt werden, grundsätzlich in jedem Anwendungsfall durch neue sterile Anlagen ausgetauscht werden. Bei der bekannten Pumpvorrichtungen ist dieser Austausch äußerst arbeitsaufwendig und kostenintensiv.

Aufgabe der Erfindung ist es, eine Pumpvorrichtung der eingangs genannten Art dahingehend zu verbessern, daß sie eine Fördermenge in genauen reproduzierbaren und kontrollierbaren Dosen problemlos abgeben kann, wobei die Förderflüssigkeit nur gering mechanisch belastet wird.

Diese Aufgabe wird durch eine Pumpvorrichtung mit den Merkmalen des Anspruchs 1 gelöst.

Die Pumpvorrichtung arbeitet nach folgendem Prinzip: Durch eine Kolbenpumpe wird dem Hohlraum eine Arbeitsflüssigkeit zu- oder abgeführt. Entsprechend bewegt sich der flexible Wandteil des Arbeitshohlraumes hin und her. Da der flexible Wandteil des Arbeitshohlraumes flächig in adhäsivem Kontakt mit dem flexiblen Wandteil der Förderkammer steht, wird diese Hin- und Herbewegung direkt auf die in der Förderkammer befindliche Förderflüssigkeit übertragen, und zwar in dem Sinne' daß Saug- bzw. Druckbewegungen entstehen. Über die als Ventile dienenden Schlauchklemmen an den Ab- und Zuflüssen aus und in die Förderkammer, erfolgen die Saug- bzw. Ausstoßbewegungen. Um ein adhäsives luftblasenfreies Aneinanderliegen der flexiblen Wandteile aneinander zu gewährleisten, sind diese mit einer Absaugvorrichtung zum Evakuieren eventuell zwischen ihnen befindlicher Luft verbindbar, wobei der Wandteil der Förderkammer auch dehnbar ist, um sich völlig glatt an den anderen Wandteil anschmiegen zu können. Somit können sich die Volumina, da die restlichen Wände der Förderkammer und des Hohlraums fest sind, nur im vorgegebenen Maße ändern. Zur Dosierung der Fördermenge wird eine Kolbenpumpe mit einstellbarer Hubmenge verwendet.

Bei der Ausführung der Pumpvorrichtung gemäß Anspruch 2 bestehen die flexiblen Wände aus ineinandergeschobenen rohrförmigen Schlauchteilen; bei der nach Anspruch 3 liegen diese flächig aufeinander.

Eine weitere vorzugsweie Ausgestaltung der Erfindung besteht darin, daß der Hohlraum, der flexible Wandteil, das feste Gehäuse und die Anschlußleitung einen mit der Kolbenpumpe verbundenen Pumpenkopf bilden, der gasdicht und lösbar mit einem aus dem flexiblen Wandteil, dem Anschlußstück und dem Zu- und Abfluß bestehenden Wegwerfteil verbunden ist.

Gemäß Anspruch 5 ist der Zwischenraum zwischen den flexiblen Teilen vorzugsweise über einen Kanal an die Absaugeinrichtung anschließbar.

Entsprechend dem Anwendungsgebiet der Pumpvorrichtung kann es vorteilhaft sein, einen Drucksensor und eine Heizung vorzusehen. Die Pumpenvorrichtung ist vorzugsweise zum Bluttransport einsetzbar.

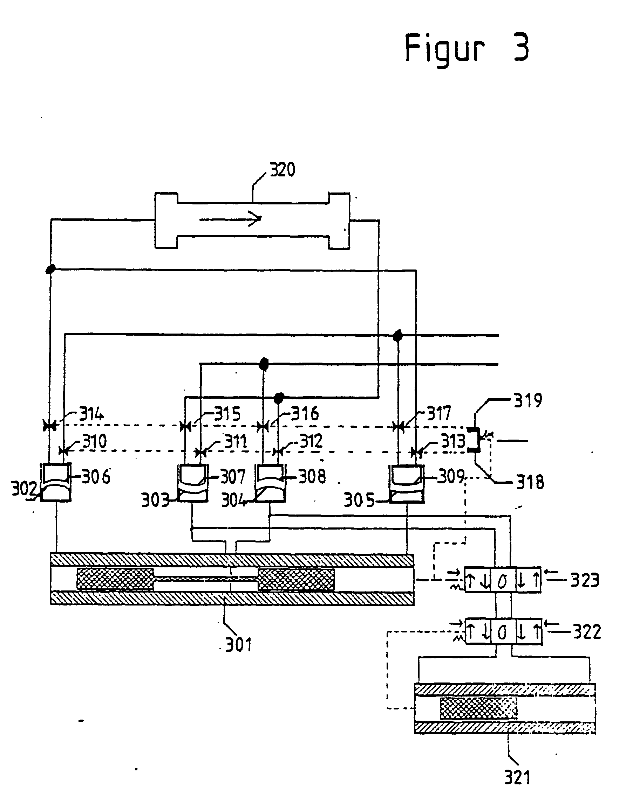

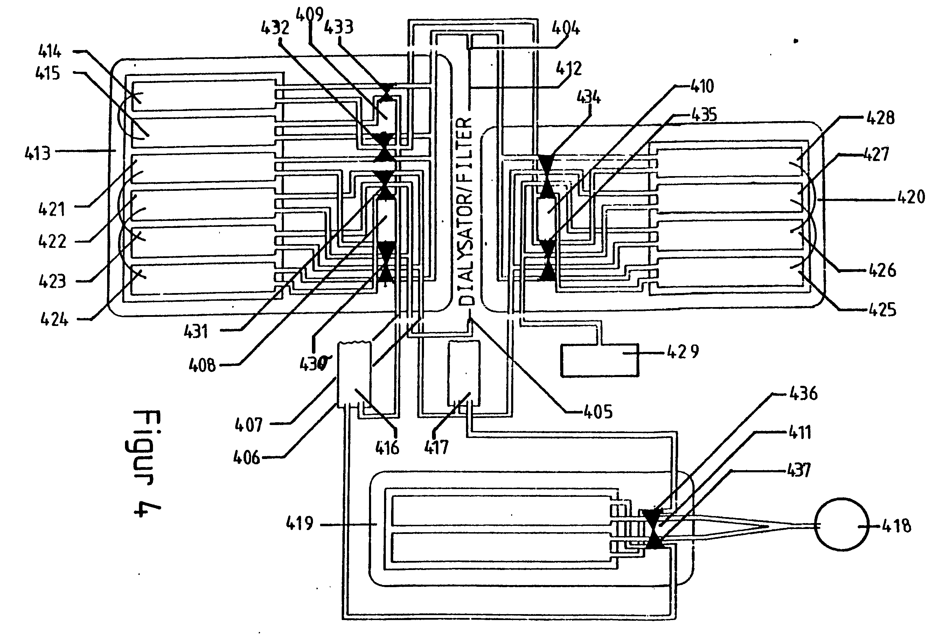

Die Vorteile der vorzugsweisen Ausgestaltung der Erfindung gemäß den Ansprüchen 8 bis 11 werden bei Erläuterung der Fig. 3 und 4 in der Figurenbeschreibung angeführt.

Nachstehend wird die Erfindung anhand von Ausführungsbeispielen unter Bezug auf Zeichnungen näher erläutert.

Es zeigt:

Fig. 1 einen Querschnitt durch eine Ausführungsvariante einer Pumpvorrichtung;

Fig. 2 einen Querschnitt durch zwei miteinander verbundenen Pumpvorrichtungen einer anderen Ausführungsvariante, die in gegenläufigen phasen arbeiten;

Fig. 3 ein antriebs- und blutseitiges Fließschema im Anwendungsfall einer Blutdosierung mit steuerbarer Ultrafiltration beim Einsatz von mehreren baugleichen Pumpvorrichtungen mit Volumendifferenzerzeugung hinter einem Dialysator;

Fig. 4 eine Zusammenfassung von drei Pumpvorrichtungen zur Durchführung einer Haemofiltration im Single-Needle-Betrieb, wobei die Volumendifferenzerzeugung vor dem Dialysator erfolgt.

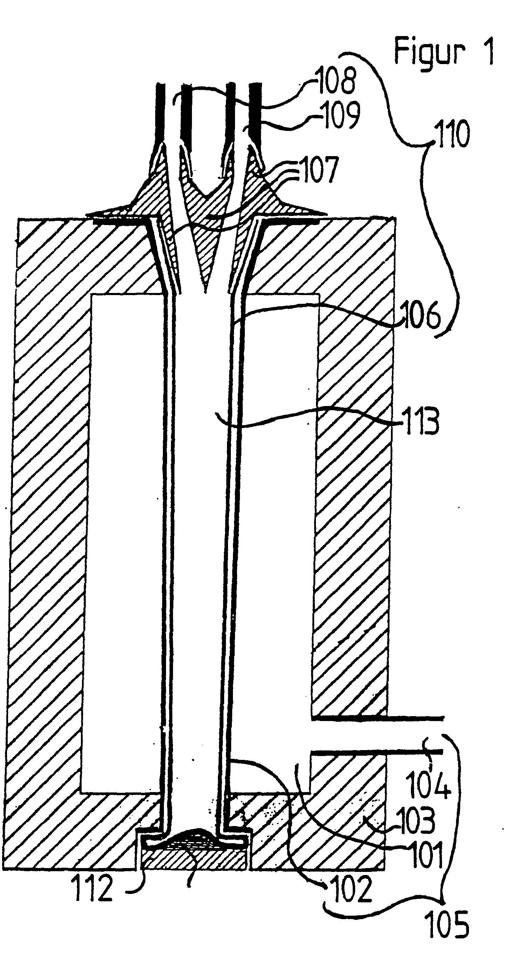

Das in Fig. 1 dargestellte Ausführungsbeispiel einer Pumpvorrichtung weist einen Hohlraum 101 auf, der von einem hohlzylindrischen flexiblen Wandteil 102 (Dauermembran) und einem festen Gehäuse l03 begrenzt wird. Der hohlzylindrische flexible Wandteil 102 ist zwischen zwei sich gegenüberliegenden Wänden in Durchbrüchen des Gehäuses 103 ortsfest und flüssigkeitsdicht angeordnet. Der Hohlraum 101 ist über eine Anschlußleitung 104 mit einer nicht dargestellten Kolbenpumpe verbunden. Im Hohlraum 101 befindet sich eine Arbeitsflüssigkeit, durch die der von der Kolbenpumpe periodisch erzeugte Über- bzw. Unterdruck in den Hohlraum 101 übertragen wird, wodurch entsprechend der flexible Wandteil 102 gewölbt bzw. zurückgezogen wird und so Pumpbewegungen ausführt. Die beschriebenen Teile bilden den Pumpkopf 105. Im hohlzylindrischen Wandteil 102 ist paßgerecht eine hohlzylindrische Pumpkammer als flexibler Wandteil 106 für die Förderflüssigkeit angeordnet. Die Pumpkammer 106 (Wechselmembran) besteht aus einem flexiblen Rohr. An einem Ende ist das Rohr mittels eines Anschlußstücks 107 ortsfest im Durchbruch des Gehäuses 103 lösbar angeordnet, das einen Zu- und Abfluß 108, 109 aufweist. Die Teile 106 bis 109 bilden einen auswechselbaren Teil 110, den Wegwerfteil 110 der Pumpvorrichtung. Das andere Ende des Rohrs ist verschlossen und mittels eines Halteelements 112 im Gehäuse 103 fest einklemmbar. Das Anschlußstück 107 und/oder das Halteelement 112 kann konisch (s. in Fig. 1 das Anschlußstück 107) oder stufenförmig gestaltet sein, wobei die Durchbrüche im Gehäuse eine entsprechende Gestalt aufweisen. Der flexible Wandteil 102 und/oder das flexible Teil 106, das Rohr der Pumpkammer, kann hierbei als Dichtelement dienen. Es besteht auch die Möglichkeit, den Zufluß in den Förderraum 113 der Pumpkammer 106 an einem Ende und den Abfluß am anderen Ende vorzusehen (nicht dargestellt). Vorzugsweise wird die Luft zwischen dem flexiblen Wandteil 102 und dem flexiblen Teil 106 der Förderkammer entfernt, so daß diese beiden Teile flächig - gegebenenfalls durch erzeugtes Vakuum - dauerhaft aufeinanderzuliegen kommen und praktisch eine Doppelwand bilden, die bei Hubbewegungen der Kolbenpumpe gleiche Wege zurücklegt, so daß die Pumpbewegungen des flexiblen Wandteils 102 störungsfrei auf die Förderflüssigkeit übertragen werden.

Wird über die Anschlußleitung 104 durch die Arbeitsflüssigkeit auf die rohrförmige flexible Wand 102 (Dauermembran) ein allseitiger Druck ausgeübt, bewegen sich deren Wände und damit auch die Wände 106 aufeinander zu und verkleinern den Innenraum. Befindet sich eine Flüssigkeit in dem durch die Wände 106 gebildeten Innenraum der Förderkammer, so wird sie daraus ausgetrieben. Hierzu sind die zwei Zu- und Abflüsse 108 bzw. 109 vorgesehen, die durch Ventile steuerbar sind. Bei einem Saugdruck erfolgt umgekehrt ein Ansaugen in die Förderkammer.

Über eine Ventilsteuerung kann je nach der Kolbenbewegung der Kolbenpumpe Druck oder Unterdruck erzeugt werden, was dann zu einem Ausstoßen und Ansaugen der Förderflüssigkeit führt.

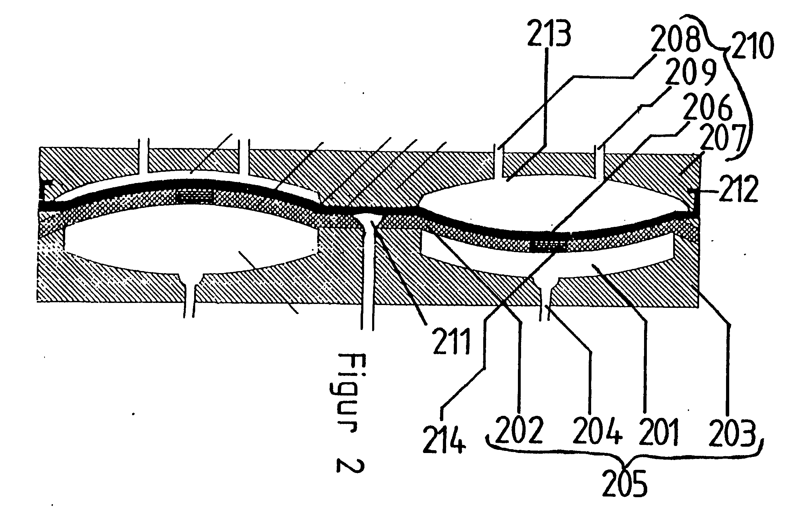

Das in Fig. 2 dargestellte, eine andere Ausführung aufweisende Ausführungsbeispiel einer Pumpvorrichtung ist als nebeneinanderliegende Doppelpumpvorrichtung gestaltet, wobei die einzelnen Pumpvorrichtungen in gegenläufigen Phasen arbeiten sollen.

Jede Pumpvorrichtung weist einen zylindrischen Hohlraum 201 auf, der von einem gewölbten, einen Deckel bildenden flexiblen Wandteil 202 (Dauermembran) und einem festen Gehäuse 203 begrenzt wird. Hierbei ist das Gehäuse 203 und auch der flexible Wandteil 202 für beide Pumpvorrichtungen in einem Stück gestaltet. Jeder Hohlraum 201 ist über eine Anschlußleitung 204 mit einer nicht dargestellten Kolbenpumpe verbunden. Der flexible Wandteil 202 ist unlösbar und flüssigkeitsdicht mit dem Gehäuse 203 auf eine beliebige bekannte Weise verbunden. Die Teile 201 bis 204 stellen eine konstruktive Einheit, den Pumpenkopf 205, dar.

Jedem flexiblen Wandteil 202 gegenüber ist ein etwa entsprechend großer flexibler Wandteil 206 einer Förderkammer 213 angeordnet. Der flexible Wandteil 206 ist einem den Förderhohlraum 213 abschließenden Anschlußgehäuse 207 flüssigkeitsdicht zugeordnet. Jede Förderkammer weist einen Zu- und Abfluß 208, 209 auf. Die Teile 206 bis 209 bilden einen auswechselbaren Teil 210, den "Wegwerfteil" der Pumpvorrichtung. Der Wegwerfteil 210 ist z.B. durch Schnellverschlüsse oder beliebige andere bekannte Verschlüsse derart auf dem Pumpenkopf 205 lösbar befestigt, daß die flexiblen Wandteile 202 des Pumpenkopfes und die flexiblen Wandteile 206 des Wegwerfteils 210 einander gegenüber liegen. Hierbei kann jeder der flexiblen Wandteile 202, 206 unmittelbar als Dichtung dienen. Zentral zwischen den beiden pumpvorrichtungen ist im Gehäuse 203 ein den flexiblen Wandteil 202 ebenfalls durchstossender Kanal 211 ausgeführt, der an eine nicht dargestellte Vakuumpumpe anschließbar ist, damit die Luft zwischen den elastischen Wandteilen 202 und 206 evakuiert werden kann, so daß deren absolut flächiges und dauerndes Aneinanderliegen gewährleistet wird und sie praktisch zu einer Wand werden.

Um das Aneinanderliegen der beiden flexiblen Wandteile zu verbessern und jeweils identische Volumen in den Hohlräumen zu gewährleisten, werden die flexiblen Wände 106 und 206, auch hochelastisch, d.h. dehnbar ausgeführt während die zugeordneten flexiblen Wände praktisch nicht dehnbar sein sollen. Der flexible Wandteil 206 des Wegwerfteils 210 kann entsprechend dem verwendeten Material durch Einlegen und Festhalten in einer umlaufenden Ringnut 212 oder auf beliebige andere Weise, z.B. durch Kleben oder Schweißen erfolgen. Es können nicht nur zwei, sondern auch je nach Verwendungszweck mehrere Pumpvorrichtungen, z.B. 4, 6 etc., zusammengefaßt werden.

Vorzugsweise eignen sich die Pumpvorrichtungen besonders zum Transport von Blut. Hierbei können sie z.B. in Dialyse- und Ultrafiltrationsvorrichtungen eingesetzt werden.

Nachstehend werden die flexiblen Wände 106 und 206 als Wechselmembranen und die flexiblen Wände 102 und 202 als Dauermembranen bezeichnet.

In Fig. 3 ist ein antriebs- und blutseitiges Fließschema im Anwendungsfall einer Blutdosierung mit steuerbarer Ultrafiltration beim Einsatz baugleicher Wechselmembraneinheiten zu Volumendifferenzerzeugung hinter dem Dialysator unter Verwendung der oben erläuterten Pumpvorrichtungen dargestellt.

Die Hauptpumpe 301, ein gedoppelter Pendelkolben, bewegt die Hydraulikflüssigkeit pulsativ vom und zum Arbeitsraum. Die dadurch bewirkte Verformung der Dauermembranen 302, 303, 304, 305 überträgt sich exakt auf die Wechselmembranen 306, 307, 308 und 309.

Die beiden jeweils aus vier Schlauchventilen 310 bis 317 bestehenden Schließregister 318 und 319 öffnen und schließen im Wechsel mit Schließüberschneidung, entsprechend der Pumpenbewegungsrichtung, so daß ein nahezu kontinuierlicher Fluß durch den Dialysator 320 gewährleistet ist und kein Filtratverlust erfolgen kann.

Eine Ultrafiltrationspumpe 321, die von der Hauptpumpe 301 arbeitsphasenunabhängig arbeitet, beaufschlagt mit einem Zusatzvolumen jeweils den Arbeitspumpraum, der in der Dialysatorentnahmephase arbeitet, so daß im Dialysator ein Blutstau mit Druckanstieg und eine daraus folgende Filtration erfolgt. Die Filtrationsmenge entspricht dem hinzudosierten Hydraulikflüssigkeitsvolumen. Gemäß der Wegeventilstellung wird dieses Volumen in der folgenden gegenläufigen Arbeitsphase wieder entzogen.

Das Wegeventil 322 hat die Aufgabe, die dauernd mit der Arbeitsphase der Ultrafiltrationspumpe 321 wechselnde Fließrichtung gleichzurichten. Das Wegeventil 323 ordnet die Sog- und Druckvolumenströme phasengerecht den Arbeitspumpräumen zu, die sich in der Dialysatorentnahmephase befinden.

Die Verwendung von Wegeventilen erübrigt sich, wenn die Ultrafiltrationspumpe hauptpumpensynchron läuft und die Steuerung der Ultrafiltrationsmenge über eine jeweilige Hubverstellung erfolgt.

In Fig.4 ist die Zusammenschaltung von drei Pumpvorrichtungen zur Durchführung einer Haemofiltration im Single-Needle-Betrieb unter Volumendifferenzerzeugung vor dem Dialysator dargestellt.

Anstatt die Filtration mit Hilfe einer Hydraulikflüssigkeitshinzudosierung zu bewirken, besteht auch die Möglichkeit, Blut vor dem Dialysator 412 einzubringen, so daß ebenfalls ein Abflußstau mit Druckanstieg entsteht, der die Filtrationsmenge bewirkt.

Hierzu ist jedoch eine Pumpvorrichtungseinheit 413 mit sechs Förderkammern erforderlich, wobei die Förderkammern 421, 422, 423 und 424 das Blut nahezu kontinuierlich und exakt volumenidentisch in den Dialysator 412 bzw. aus dem Dialysator 412 fördern. Die Förderkammern 414 und 415 dosieren die Menge Blut hinzu, die der beabsichtigten Filtrationsmenge entspricht. (Die Bindeklammern hinter den Pumpenkammern weisen auf die jeweils gegenläufige Arbeitsrichtung hin).

Bei dieser Anwendung wird das Blut der arteriellen Depotkammer 416 entnommen, bzw. es wird in die venöse Depotkammer 417 gefördert, bevor es zum Patienten 418 zurückgeführt wird.

Eine nach dem Single-Needle-System arbeitende Blutpumpe 419 bewirkt intermittierend die Blutentnahme vom Patienten bzw. die Rückführung dorthin. Im Gegensatz zur herkömmlichen Funktionsweise eines Single-Needle-Systems ist es somit möglich, eine nahezu kontinuierliche Blutpassage durch den Dialysator 412 oder Filter zu gewährleisten, trotz diskontinuierlicher An- und Abförderung.

Die Pumpvorrichtungseinheit 420 ist im wesentlichen baugleich mit den Pumpkammern 421 bis 424 der Pumpvorrichtungseinheit 413. Sie dient der Durchführung einer volumetrischen Haemofiltration. Dabei dosieren die Förderkammern 425 und 428 Blut in den Dialysator 412. Da es nicht abgefördert wird, entspricht es der beabsichtigten Filtrationsmenge. Die Förderkammern 426 und 427 fördern gleichzeitig und volumenidentisch Substitutionslösung aus einem Gefäß 429 in die venöse Depotkammer 417, so daß die Gesamtbilanz für den Patienten wieder ausgeglichen ist.

Je nach Verwendungsart sind die Förderkammern der Einheiten als Doppel-, Vierfach- oder Sechsfachpumpvorrichtungseinheiten zusammengefaßt, so daß der Aufwand des Einbringens auf einen Handgriff beschränkt bleibt. Die zu- bzw. abführenden Blutschlauchgruppen sind so miteinander verbunden, daß der Anwender nur wie bei herkömmlichen Systemen die dialysator- 404 und 405 und patienten seitigen 406 und 407 Schläuche anzuschließen braucht. Bedingt durch die Gestaltung der Blutwege bleiben Aussparungen 408, 409, 410 und 411 frei, durch die die Steuerelemente für die Schlauchventile 430 bis 437 ragen, durch die entweder die Zu- oder die Abflüsse freigegeben werden.

Kommt die Flachmembranausführung gemäß Fig. 2 zur Anwendung, so kann auf externe Blutwegeverflechtung gänzlich verzichtet werden, wenn ein Zwischenflansch verwendet wird, der mit Auslassungen für die Bewegungszonen von Dauer- und Wechselmembran versehen ist. Auf diesem Zwischenflansch, der als Auflagefläche für die Wechselmembraneinheit dient, ist die Blutwegeverflechtung in der Weise eingefräst, daß sich die Wechselmembran beim Einbringen von Unterdruck in diese Ausfräsungen anformt. Dort können die Blutwege dann auch von nach oben ausrückenden Ventilkeilen geöffnet bzw. geschlossen werden. Die starre Deckelplatte 207 stellt hierbei das Widerlager dar. Die vier erforderlichen Anschlußstutzen für die Dialysator- bzw. Patientenschläuche können also direkt an die Abgangsstutzen des Pumpendeckels angeschlossen werden.

Aus Sicherheits- und Überwachungsgründen ist es erforderlich, den im Innenraum der Wechselmembraneinheit entstehenden Druck zu erfassen. Es sind deshalb formstabile Drucksensoren (114 Fig. 1; 214 Fig. 2) vorzusehen. Sowohl bei der Hohlkörper- wie auch bei der Flachmembranausführung kann der Sensor aber auch im Hydraulikflüssigkeitsraum oder im entsprechenden Zuführungsanschluß eingebracht sein. Ebenso kann er sich direkt, unter Luftausschluß, an der Wechselmembran befinden, indem er bei der Hohlkörperausführung beispielsweise in das Halteelement 112 eingearbeitet ist, bzw. bei der Flachmembranausführung Bestandteil der Dauermembran 202 ist und flächenbündig mit dieser in Verbindung zur Wechselmembran 206 steht.

Kommt es zur Membranruptur, so bricht der vorhandene Unterdruck plötzlich zusammen, indem er das Blut über den Kanal 211 aus der Pumpkammer 213 absaugt, die Wechselmembran 206 löst sich von der Dauermembran 202 und somit auch von dem in die Dauermembran eingearbeiteten Drucksensor 214. Der Drucksensor registriert kurzfristig starke Druckschwankungen, so daß bei entsprechender Signalverarbeitung ein Alarm und die Einleitung der automatischen Sicherheitsfunktionen eingeleitet werden kann.

In bestimmten Fällen (Haemofiltration, Diafiltration usw.) kann es erforderlich sein, den Wärmeverlust des Blutes auszugleichen. Handelt es sich materialmäßig um eine entsprechende Dauermembran, so kann sie mit Widerstandsheizelementen versehen werden. Ebenso kann jedoch auch die Hydraulikflüssigkeit selbst beheizt werden, so daß ein Wärmeübergang auf die Dauer- und Wechselmembran stattfindet.

Beim Einsatz des Verfahrens adhaesive Membrantechnik Blutfeindosierung ergeben sich deutliche Vorteile, die mit herkömmlichen Blutpumpensystemen unter vertretbarem technischen und wirtschaftlichen Aufwand nicht erreicht werden konnten. Im Wesentlichen sind dies:

1. Bluttransport mit Feindosierpräzision für den Routinebetrieb.

2. Eine Pumpcharakteristik, die der des menschlichen Herzens entspricht.

3. Das extracorporale Blutvolumen wird kleiner als bei Verwendung herkömmlicher Blutschlauchsysteme.

4. Der Pumpvorgang erfolgt berührungsfrei, so daß die mechanische Belastung für das Blut sicher vermieden wird. Schlauchabrieb ist nicht möglich.

5. Kontinuisierung der Membranpassagenflußgeschwindigkeit im Dialysator, im Gegensatz zum intermittierenden Fluß bei Verwendung herkömmlicher Single-Needle-Systeme.

6. Kontinuisierung des blutseitigen Transmembrandrucks und somit zuverlässige Transmembrandrucksteuerung im Gegensatz zu unvermeidlich hohen Transmembrandruck-Amplituden bei Verwendung herkömmlicher Single-Needle-Systeme.

7. Volumetrische Single-Needle-Betriebs-Steuerung.

8. Präzise Erfassung der Druckverhältnisse über die gesamte Strecke Pumpenausgang/Kanüle bzw. Kanüle/Pumpenausgang und daher mit geringem Aufwand die Möglichkeit shuntkapazitätsorientiert zu fördern.

9. Clottingüberwachung aufgrund des Druckamplitudenverhaltens vor und hinter dem Filter.

10. Präzise Erfassung und Steuerung des jeweiligen Schlagvolumens, woraus sich die quantitative Definitionsmöglichkeit und Verminderung der Rezirkulationsvolumina ergibt.

11. Blutseitige Entzugsratensteuerung durch Blutvolumenhinzudosierung.

12. Blutseitige Entzugsratensteuerung durch Verminderung des Blutvolumenentzugs aus dem Dialysator.

13. Volumetrische Haemofiltration.

**Patentansprüche**

1. Pumpvorrichtung zur Förderung von gegen mechanische Beanspruchungen hochempfindlichen Flüssigkeiten, mit einem festen Gehäuse (103; 203), das einen flexiblen Wandteil (102; 202) aufweist und einen abgedichteten Hohlraum (101; 201) veränderlichen Volumens umgibt, der eine Arbeitsflüssigkeit enthält und über eine Anschlußleitung (104; 204) mit einer Pumpe verbunden ist, und mit einer flüssigkeitsdichten, mit Zu- und Abflüssen (108, 109; 208, 209) mit Ventilen versehenen Förderkammer für die Förderflüssigkeit, die mindestens einen flexiblen Wandteil (106; 206) aufweist, wobei die flexiblen Wände (106; 206) des Gehäuses und der Förderkammer einander gegenüberliegend in flächigen Berührungskontakt bringbar angeordnet sind und als gegenseitige luftdichte Dichtungen gestaltet sind, dadurch gekennzeichnet, daß die Pumpe eine Kolbenpumpe mit einstellbarer Hubmenge ist, daß die Ventile steuerbare Schlauchklemmen sind, daß der flexible Wandteil (106; 206) der Förderkammer auch dehnbar ist und daß die Luft im Zwischenraum zwischen den flexiblen Wandteilen (102 und 106; 202 und 206) durch eine Absaugeinrichtung entfernbar ist.

2. Pumpvorrichtung nach Anspruch 1, dadurch gekennzeichnet, daß die flexiblen Teile (102; 106) des Gehäuses (103) und der Förderkammer jeweils hohlzylindrisch ausgeführt sind und die Förderkammer innerhalb des hohlzylindrischen Teils (102) des Gehäuses (103) angeordnet ist.

3. Pumpvorrichtung nach Anspruch 1, dadurch gekennzeichnet, daß die flexiblen Teile (202; 206) des Gehäuses (203) und der Förderkammer flach ausgeführt sind.

4. Pumpvorrichtung nach einem der Ansprüche 1 bis 3, dadurch gekennzeichnet, daß der Hohlraum (101; 201), der flexible Wandteil (102; 202), das feste Gehäuse (103; 203) und die Anschlußleitung (104; 204) einen mit der Kolbenpumpe verbundenen Pumpenkopf (105; 205) bilden, der gasdicht und lösbar mit einem aus dem flexiblen Wandteil (106; 206), dem Anschlußstück (107; 207) und dem Zu- und Abfluß (108, 109; 208, 209) bestehenden Wegwerfteil (110; 210) verbunden ist.

5. Pumpvorrichtung nach einem der Ansprüche 1 bis 4, dadurch gekennzeichnet, daß der Zwischenraum zwischen den flexiblen Teilen (202; 206) über einen Kanal (211) an die Absaugeinrichtung anschließbar ist.

6. Pumpvorrichtung nach einem der Ansprüche 1 bis 5, dadurch gekennzeichnet, daß ein Drucksensor und gegebenenfalls eine Heizung vorgesehen sind.

7. Pumpvorrichtung nach einem der Ansprüche 1 bis 6, dadurch gekennzeichnet, daß die Förderflüssigkeit Blut ist.

8. Pumpvorrichtung nach einem der Ansprüche 1 bis 7, dadurch gekennzeichnet, daß mehrere Pumpvorrichtungen derart angeordnet und von Kolbenpumpen mit Arbeitsflüssigkeit versorgt werden, daß gleichzeitig durch eine Anzahl von Pumpvorrichtungen die gleiche Volumenmenge an Förderflüssigkeit angesaugt und durch die gleiche Anzahl von Pumpvorrichtungen wieder abgegeben wird.

9. Pumpvorrichtung nach einem der Ansprüche 1 bis 8, dadurch gekennzeichnet, daß die Schlauchklemmen der Zu- und Abflüsse (108, 109; 208, 209) steuerbar sind und die Zu- und Abflüsse (108, 109; 208, 209) mit einer Blutbehandlungsvorrichtung, wie beispielsweise für die Dialyse und/oder für die Ultrafiltration verbunden sind.

10. Pumpvorrichtung nach einem der Ansprüche 1 bis 9, dadurch gekennzeichnet, daß mindestens eine zusätzliche Kolbenpumpe zur Dosierung eines Störvolumens vor oder nach einer Blutbehandlungsvorrichtung für die Arbeitsflüssigkeit vorgesehen ist.

11. Pumpvorrichtung nach einem der Ansprüche 1 bis 9, dadurch gekennzeichnet, daß mindestens eine zusätzliche Kolbenpumpe und mindestens eine zusätzliche Förderkammer zur Dosierung eines Störvolumens vor oder nach einer Blutbehandlungsvorrichtung für die Förderflüssigkeit vorgesehen sind.

**Revendications**

1. Dispositif de pompage servant à pomper des liquides très sensibles à des contraintes mécaniques, comportant un carter fixe (103; 203), qui possède un élément de paroi flexible (102; 202) et entoure une cavité (101; 201) de volume variable, fermée de façon étanche, qui contient un liquide de travail et est reliée à une pompe par l'intermédiaire d'une canalisation de raccordement (104; 204), et une chambre d'entraînement étanche aux liquides, prévue pour le liquide d'entraînement et qui comporte des accès d'entrée et de sortie (108, 109; 208, 209) équipés de soupapes et possède au moins un élément de paroi flexible (106; 206), les parois flexibles (106; 206) du carter et de la chambre d'entraînement étant disposées de manière à pouvoir être amenées en vis-à-vis en étant réciproquement en contact à plat l'une sur l'autre et étant agencées à la manière de garnitures d'étanchéité opposées, étanches à l'air, caractérisé en ce que la pompe est une pompe à piston possédant une cylindrée réglable, que les soupapes sont des pinces pour tuyaux, commandables, que l'élément de paroi flexible (106; 206) de la chambre d'entraînement peut être également dilaté et que l'air situé dans l'espace intercalaire compris entre les éléments de paroi flexibles (102 et 106; 202 et 206) peut être évacué au moyen d'un dispositif d'aspiration.

2. Dispositif de pompage selon la revendication 1, caractérisé en ce que les éléments flexibles (102; 106) du carter (103) et de la chambre d'entraînement sont réalisés respectivement sous la forme de cylindres creux et que la chambre d'entraînement est disposée à l'intérieur de la partie cylindrique creuse (102) du carter (103).

3. Dispositif de pompage selon la revendication 1, caractérisé en ce que les éléments flexibles (202; 206) du carter (203) et de la chambre d'entraînement sont plats.

4. Dispositif de pompage selon l'une des revendications 1 à 3, caractérisé en ce que la cavité (101; 201), l'élément de paroi flexible (102; 202), le carter rigide (103; 203) et la canalisation de raccordement (104; 204) forment une tête de pompe (105; 205), qui est reliée à la pompe à piston et est raccordée de façon amovible et d'une manière étanche aux gaz à un élément jetable (110; 210) constitué par l'élément de paroi flexible (106; 206), l'élément de raccordement (107; 207) et les accès d'entrée et d'évacuation (108, 109; 208, 209).

5. Dispositif de pompage selon l'une des revendications 1 à 4, caractérisé en ce que l'espace intercalaire compris entre les éléments flexibles (202; 206) peut être raccordé, par l'intermédiaire d'un canal (211), au dispositif d'aspiration.

6. Dispositif de pompage selon l'une des revendications 1 à 5, caractérisé en ce qu'il est prévu un capteur de pression et éventuellement un dispositif de chauffage.

7. Dispositif de pompage selon l'une des revendications 1 à 6, caractérisé en ce que le liquide entraîné est du sang.

8. Dispositif de pompage selon l'une des revendications 1 à 7, caractérisé en ce qu'il est prévu plusieurs dispositifs de pompage, qui sont alimentés avec un liquide de travail par des pompes à piston et que le même volume de liquide entraîné est aspiré simultanément par un certain nombre de dispositifs de pompage et est à nouveau évacué par le même nombre de dispositifs de pompage.

9. Dispositif de pompage selon l'une des revendications 1 à 8, caractérisé en ce que les pinces pour tuyaux des accès d'entrée et d'évacuation (108, 109; 208, 209) sont commandables et que les accès d'entrée d'évacuation (108, 109; 208, 209) sont reliés à un dispositif de traitement du sang, par exemple pour la dialyse et/ou l'ultrafiltration.

10. Dispositif de pompage selon l'une des revendications 1 à 9, caractérisé en ce qu'il est prévu au moins une pompe à piston supplémentaire servant à doser un volume parasite en amont ou en aval d'un dispositif de traitement du sang, pour le liquide de travail.

11. Dispositif de pompage selon l'une des revendications 1 à 9, caractérisé en ce qu'il est prévu au moins une pompe à piston supplémentaire et au moins une chambre d'entraînement supplémentaire pour le dosage d'un volume parasite en amont ou en aval d'un dispositif de traitement du sang, pour le liquide entraîné.

## Claims

1. Pump device for conveying fluids which are highly sensitive to mechanical stresses, with a fixed housing (103; 203) which comprises a flexible wall part (102; 202) and surrounds a sealed-off cavity (101; 201) of variable volume which contains a working fluid and is connected by way of an attachment conduit (104; 204) with a pump, and with a fluid-tight conveyor chamber, provided with inlets and outlets (108, 109; 208, 209) with valves, for the conveyed fluid, which comprises at least one flexible wall part (106, 206), where the flexible walls (106; 206) of the housing and of the conveyor chamber, lying opposite to one another, are bringable into flat contact and are formed as mutual airtight seals, characterised in that the pump is a piston pump with adjustable swept volume, in that the valves are controllable hose clips, in that the flexible wall part (106; 206) of the conveyor chamber is also stretchable and in that the air in the interspace between the flexible wall parts (102 and 106; 202 and 206) can be removed by a suction device.

2. Pump device according to Claim 1, characterised in that the flexible parts (102; 106) of the housing (103) and of the conveyor chamber are each made hollow-cylindrical and the conveyor chamber is arranged within the hollow-cylindrical part (102) of the housing (103).

3. Pump device according to Claim 1, characterised in that the flexible parts (202; 206) of the housing (203) and of the conveyor chamber are made flat.

4. Pump device according to one of Claims 1 to 3, characterised in that the cavity (101; 201), the flexible wall part (102; 202), the fixed housing (103; 203) and the connecting conduit (104; 204) form a pump head (105; 205) connected with the piston pump, which head is gas-tightly and detachably connected with a throw-away part (110; 210) consisting of the flexible wall part (106; 206), the attachment piece (107; 207) and the inlet and outlet (108, 109; 208, 209).

5. Pump device according to one of Claims 1 to 4, characterised in that the interspace between the flexible parts (202; 206) is connectable through a passage (211) to the suction device.

6. Pump device according to one of Claims 1 to 5, characterised in that a pressure sensor and possibly a heating system are provided.

7. Pump device according to one of Claims 1 to 6, characterised in that the conveyed fluid is blood.

8. Pump device according to one of Claims 1 to 7, characterised in that several pump devices are so arranged and supplied with working fluid by piston pumps that the same quantity by volume of conveyed fluid is sucked in at the same time by a number of pump devices and delivered again by the same number of pump devices.

9. Pump device according to one of Claims 1 to 8, characterised in that the hose clips of the inlets and outlets (108, 109; 208, 209) are controllable and the inlets and outlets (108, 109; 208, 209) are connected with a blood treatment device, for example for dialysis and/or for ultra filtration.

10. Pump device according to one of Claims 1 to 9, characterised in that at least one additional piston pump is provided for the quantity regulation of an interference volume before or after a blood treatment device for the working fluid.

11. Pump device according to one of Claims 1 to 9, characterised in that at least one additional piston pump and at least one additional delivery chamber for the quantity regulation of an interference volume before or after a blood treatment device for the conveyed fluid are provided.

Figur 1

Figur 2

Figur 3

Figur 4